## Europäisches Patentamt
## European Patent Office
## Office européen des brevets

(11) Publication number: **0 033 666**
A1

# EUROPEAN PATENT APPLICATION

(21) Application number: **81300474.4**

(22) Date of filing: **04.02.81**

(51) Int. Cl.³: **F 04 B 43/12**

(30) Priority: **04.02.80 GB 8003625**

(43) Date of publication of application:
**12.08.81 Bulletin 81/32**

(84) Designated Contracting States:
**AT BE CH DE FR IT LI LU NL SE**

(71) Applicant: **Lenton, Douglas Francis**
**58 Holmwood Road**
**Cheam Surrey SM2 7JP(GB)**

(72) Inventor: **Lenton, Douglas Francis**
**58 Holmwood Road**
**Cheam Surrey SM2 7JP(GB)**

(74) Representative: **Warren, Keith Stanley et al,**
**Baron & Warren 16 Kensington Square**
**London W8 5HL(GB)**

(54) Peristaltic pump.

(57) The pump unit comprises a housing (1) having a roller head (22) releasably fastened to the top (21) of the housing (1) and an arcuate tube retainer (26) for urging the pump tube (25) into engagement with the head (22). The retainer (26) is pivotally mounted on the housing (1) so as to be swingable between open and closed positions to permit changing of the pump tube (25) and is lockable in its closed position by a manually operated latch (81). The head (22) is rotated by a variable speed drive mechanism, including DC printed circuit motor (100) disposed within the housing (1). The retainer (26) has a removable arcuate liner (87) for engaging and urging a pump tube (25) of predetermined diameter into correct operative engagement with the head (22) and this liner (87) is readily interchangeable with other liners (87) which permit pump tubes (25) of different predetermined diameters to be used with the head (22). Also, the head (22) is interchangeable with another paediatric head (22) having a reduction gear mechanism (62) or providing a different speed ratio.

Fig.2

EP 0 033 666 A1

—1—

## IMPROVEMENTS IN PERISTALTIC PUMPS

The present invention relates to peristaltic pumps and, more particularly, although not exclusively, to such pumps for medical and laboratory purposes.

Peristaltic pumps basically comprise a rotatable pump head including a plurality of rollers rotatable about axes substantially parallel to the axis of rotation of the head, a drive mechanism for rotating the head, and a tube retainer having an arcuate surface closely adjacent and generally concentric with the head for urging a resilient pump tube into operative engagement with the head. Rotation of the head successively rotates the rollers into compressive engagement with the tube to pump fluid along the latter. Hitherto, peristaltic pumps used for medical and laboratory purposes, such as, for performing transfusions, infusions or perfusions, have been driven by relatively large synchronous electrical motors or electrical stepping motors assembled in a single unit with the pumping mechanism, that is, the head and the retainer. These relatively large motors require a 240 volt mains supply and an extensive gear mechanism connecting the motor to the pump head in order to produce the necessary reduction in the transmission ratio to achieve the small volume flows required for medical purposes. Moreover, each pumping mechanism is normally adapted to operate solely in conjunction with a single one of the several different standard sizes of resilient tubes used in peristaltic pumps and the pumping rate is therefore only variable over a limited range dependent on the speed variation of the motor. The tube is a dedicated component of the pump and, in use, its opposite ends are connected respectively to suitable giving and receiving sets. It may be removable for sterilisation purposes and, upon initial fitting or replacement of the tube, the pump has to be calibrated by adjustment of the tube retainer. Such calibration requires the services of a skilled technician and is a time-consuming procedure. It has also been found that the calibration may not be maintained over a lengthy period of operation of the pump.

In a surgical operation, a peristaltic pump tube may only be used for one procedure and, during an operation, it is often necessary to change pump tubes and/or use tubes of different diameters to provide for different ranges of flow rates. Medical giving sets, such as, intravenous giving sets in which the dispensing tube is integral with the blood bag, are available in several different sizes established to an international standard.

For medical and laboratory purposes, it is desirable to have a peristaltic pump unit in which the pump tube can readily be replaced, thereby enabling a tube of a giving set to be used as the pump tube, and to have a single pump unit for pumping fluids over a wide range of different flow rates, for example, for performing perfusions on adults at one end of the range and babies at the opposite end. With known peristaltic pumps, such a wide range presently necessitates the use of several, differently-rated, pump units. Other disadvantages of existing pump units are that the pump tubes are not readily changeable and the units are of limited flexibility and bulky. Because of their bulk and requirement for an electrical mains supply, they are not readily portable or transportable and are therefore unsatisfactory for use in transportable equipment, such as, in ambulances. A further disadvantage of existing pumps is that, in surgical procedures, in order to prevent embolism occurring in a patient, the discharge ends of the pump tubes must be visually and constantly inspected for foreign bodies in the fluids being delivered to the patients.

An object of the present invention is to provide a peristaltic pump unit which alleviates the disadvantages of hitherto known pumps of this type and which may be constructed as a relatively lightweight and readily portable article.

The invention consists in a peristaltic pump unit wherein the tube retainer is pivotally mounted adjacent one end of its arcuate surface and is swingable between an open position, in which the pump tube is releasable from the head, and a predetermined closed position, in which the arcuate surface urges the tube into operative engagement with the pump head, and wherein means is provided for indexing and locking the retainer in its predetermined closed position.

Conveniently, the indexing and locking means is constituted by a latch device which is preferably disposed adjacent the end of the arcuate surface opposite to the pivot mounting of the retainer and comprises a latch member mounted on the retainer and a cooperating detent disposed adjacent the head in a precisely predetermined position. The invention permits the pump tube to be readily changed and, when the retainer is closed after changing of the tube, its arcuate, tube-engaging surface is automatically reset in a precisely predetermined position with respect to the head and is lockable in this position by the latch device. Hence, the changing operation can be performed by any competent person and does not require the services of a skilled technician, as hitherto.

Preferably, the arcuate surface of the retainer is defined by a removable liner. This liner is mounted in the retainer for urging a tube of predetermined diameter into operative engagement with the head and is interchangeable with at least one other liner for use with a resilient tube of different predetermined diameter. Such liners enable the pump to be used with different size

-4-

tubes, whereby it is adapted to pump precisely controlled amounts of fluid over a wider range of fluid flow rates than has hitherto been achieved with existing peristaltic pump units. The retainer and liner may be plastics mouldings.

In the medical field, three standard diameters of tube are used internationally with peristaltic pumps for the purposes of supplying or withdrawing fluids from the adult human body, and the tube holder may be adapted to be fitted with any of three different liners for use with these three different standard diameters of tube and for urging the selected tube into engagement with the rollers of the pump head. The retainer may also be fitted with a further liner which is adapted to urge a double, resilient, tube into engagement with the rollers so that the pump unit may be used for simultaneously supplying and withdrawing fluids.

Preferably, the standard pump head is removable and is interchangeable with another, reduction head which may have the same number of rollers as the standard head but incorporates a reduction gear mechanism to reduce its speed of rotation. This reduction head, in conjunction with a further range of interchangeable liners, permits use of the pump unit with smaller tube diameters suitable, for example, for paediatric purposes.

For special purposes, such as heart operations, a special pump head may be installed on the pump unit for use with a double tube for simultaneously supplying and withdrawing fluid. This special head may comprise two rows of rollers with one row offset or advanced with respect to the other row by approximately 20%, whereby the pump unit can be operated to supply and withdraw blood in a manner corresponding more physiologically to the pumping action of the human heart.

Conveniently, the drive mechanism comprises a DC printed circuit motor coupled to the pump head by a reduction gear mechanism. Such a motor is relatively small and may only require a 12 v DC supply, such as is obtainable from a normal automobile battery, so that the pump unit is readily portable and usable in the field. The printed circuit motor may be arranged to be energized using pulse drive techniques to control its speed, that is, by applying electrical potential for a longer or shorter mark-space ratio, depending on the load imposed on the motor and the desired rotational speed. The reduction gear mechanism may comprise a planetary gear system comprising one or more planetary gear members mounted on a carrier member rotatable by the motor, the or each planetary gear member comprising a first gear wheel or pinion meshing with a fixed gear ring, and a second gear wheel or pinion rotatable with the first gear and meshing with a rotatable gear ring coupled to the pump head.

Electrical control circuitry for the pump unit may include means for adjusting the speed of the drive mechanism so as to enable the pumping rate to be precisely controlled over a wide range for any selected tube diameter. The circuitry may also include means for providing a visual indication of the pumping rate at which the pump unit is operating. This visual indication may be derived from a signal identifying the speed of rotation of the drive mechanism or pump head and another signal identifying the diameter of the tube cooperating with the head. The signal identifying the driving speed may be produced by means, such as a photo-tachometer device, for sensing the rotational speed of the motor, whilst the tube identifying signal may be produced by means for sensing the selected liner fitted into the tube retainer. It has been found that, for a selected tube diameter, the ratio of motor speed to pumping rate is non-linear and may vary by approximately 20% over the speed range of the drive mechanism owing to increased pumping efficiency at higher rotational speeds of the pump head and, preferably, an automatic correction circuit is included in the control circuitry to compensate for this non-linear variation.

It is desirable that the control circuitry include fault-detection or safety devices for monitoring the operation of the unit and for producing a warning signal and/or switching-off the drive mechanism in the event of a fault occurring in the unit or an irregularity being detected in the fluid being pumped by the unit. The warning signal may be produced by an audio and/or visual alarm device. The safety devices may include means for switching-off the drive motor in the event of the motor overspeeding or failure of the motor speed measuring means, and the alarm device may be arranged to produce a warning signal when the motor has been so switched-off. Additionally, a photodetector device may be arranged to scan the fluid flow through the tube at the discharge side of the pump in order to detect bubbles or other foreign matter, such as air bubbles or blood clots in a blood flow, and switch-off the motor and actuate the alarm device in response to detecting such contaminants. The circuitry may also include means for manually overriding the safety devices so that the motor can be re-started, after it has been stopped in response to detection of a fault or contaminant in the fluid flow and even if the fault is not rectified or the contaminant removed, when it has been determined that there is no danger in continuing the pumping operation.

In order that the present invention may be more readily understood, reference will now be made to the accompanying drawings in which:-

Fig. 1 is a perspective view of one embodiment of the invention showing the tube retainer in its closed position,

Fig. 2 is a perspective view similar to Fig. 1 showing the tube retainer in its open position,

Fig. 3 is a front elevation of the pump unit, showing the tube retainer in its open position and the pump head removed,

Fig. 4 is a section along the line IV-IV of Fig. 1 with the pump head removed,

Fig. 5 is a side elevation illustrating a partial section along the line V-V of Fig. 2 and with the pump head removed,

Fig. 6 is an axial section through a standard pump head,

Fig. 7 is an axial section through a paediatric pump head,

Fig. 8 is a fragmentary perspective view illustrating the tube retainer and a liner,

Fig. 9 is a block circuit diagram of the motor speed control and alarm circuits of the pump unit, and

Fig. 10 is a block circuit diagram of the display logic.

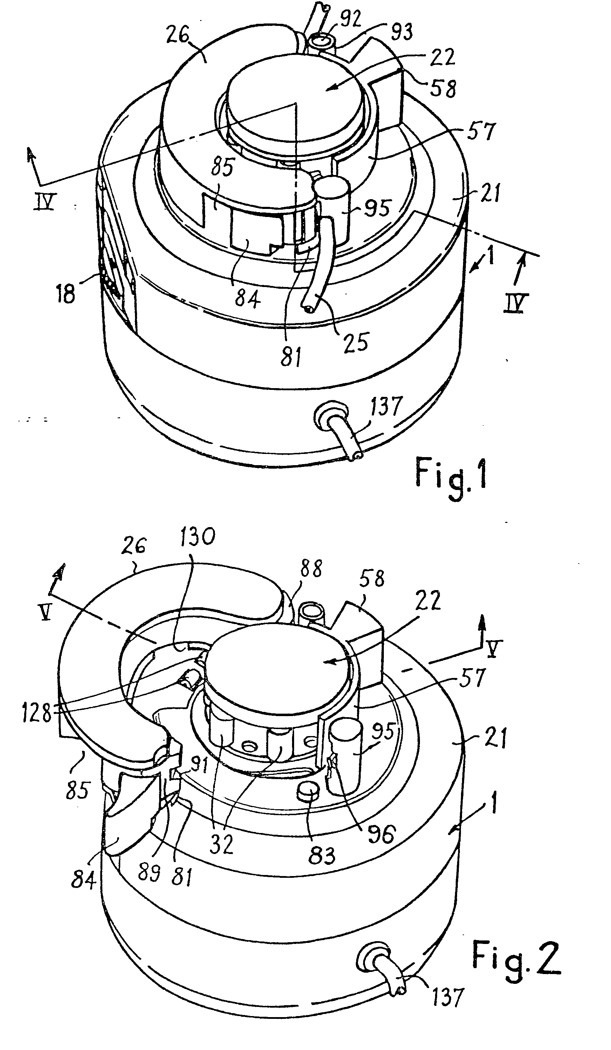

Referring to Figs. 1 to 4 of the accompanying drawings, the pump unit comprises a generally cylindrical housing 1 constructed in three parts 2,3,4. The top and bottom parts 2,4 are plastics mouldings whilst the central part 3 is formed from metal and serves as a heat sink. The bottom moulding 4 includes an upwardly projecting skirt 5 surrounding the central metal part 3 so as to insulate the latter from external contact. It is attached to the central part by three screws 6 and associated tubular spacers 7 equally spaced about the housing, whilst the central part is attached to the top part 2 of the housing by four screws 8 equally spaced about the housing and screwing into studs 9 which are extensions of pegs 10 projecting downwardly from the top part 2. These screws, studs and pegs 6, 8, 9, 10 also serve as locating and fixing points for internal parts of the pump unit, as will hereinafter be more fully described.

At one side, the top part 2 of the housing is formed with an opening 12 in which is disposed a digital display panel 13 indicating the rate of flow of the liquid being pumped by the pump unit. Also disposed in this opening, at opposite sides of the display panel 13, are start and stop buttons 14,15, a test button 16 and a surge button 17. Projecting through the bottom of the opening is a portion of a control wheel 18 which adjusts the setting of a potentiometer 19 mounted within the housing and controlling the rotational speed of the drive motor, as will hereinafter be more fully described.

Mounted centrally on the top 21 of the housing 1 is a rotatable pump or roller head 22 which is coupled to a drive shaft 23 projecting through an opening 24 in the top part of the housing, and includes rollers 32. The rollers 32 rotate in engagement with a resilient pump tube 25, which is held against the rollers by an arcuate tube retainer 26, in order to pump liquid along the tube. The head 22 is adapted to be readily changeable and is secured to the top of the housing by means of a bayonet fastening which includes a metal ring 27, constituting the female part of the fastening, secured to the top of the housing coaxially with the shaft 23 in a rebate formed about the periphery of a circular recess 28 in the housing top. The opening 24 is formed in a tapered boss 29 which projects upwardly from the bottom of the recess 28 and provides a guide taper for facilitating attachment of the head 22 to the housing and coupling of the drive shaft 23 to the head. A suitable thermal connection (not shown) is provided between the metal heat sink part 3 of the housing and the metal bayonet ring 27 to permit dissipation of heat through the pump head.

For medical purposes, the pump may be provided with two changeable heads, namely, a standard head, as shown in Fig. 6, which is suitable for use with the standard diameters of tube used internationally for supplying and withdrawing fluids from the adult human body, and a paediatric head, as shown in Fig. 7, which is used with the smaller standard tube diameters employed for paediatric purposes. It has been found that optimum results are achieved with a head 22 having five rollers equally spaced about the head tangentially to a circumscribed circle having a diameter not greater than 63.5 mm ($2\frac{1}{2}$ inches) and preferably of approximately 51 mm (2 inches), the rollers having a diameter of approximately 12.5 mm ($\frac{1}{2}$ inch). When pumping blood, it has been found that this configuration is capable of pumping blood without rupturing red blood cells.

Referring particularly to Fig. 6, the standard head comprises a roller cage 30 which is rotatably mounted on a hollow hub 31 and supports five freely rotatable rollers 32. The cage 30 comprises upper and lower radial flanges 33,34 projecting from a central sleeve 35 and is suspended from a cage cap 36. The sleeve 35 is rotatably mounted on the hub 31 by a plain bearing 37 whilst the cap 36 is rotatably supported on the upper end of the hub by a thrust washer 38. The cap has a central input shaft 39 integral therewith. This input shaft projects downwardly through the hollow hub 31 and has a slot 40 at its lower end for engaging with a driving blade 41 at the top of the drive shaft 23. The lower end of the input shaft 39 has a reinforcing sleeve 42 and is located in position by a suitable washer and circlip assembly 43 on the shaft 39 and a cooperating annular shoulder 44 formed on the hub. The lower end 45 of the hub 31 is tapered outwardly so that its internal surface 46 has a complementary taper to that of the boss 29 on the top of the housing and cooperates with this tapered boss to guide the head into its correct position on the housing. The cap is connected to the roller cage 30 by screws 47 which are screwed through the upper flange 33 of the cage.

The rollers 32 are rotatably mounted on hollow shafts 48 which have opposite ends located in aligned apertures 49 in the flanges 33,34 of the cage and are secured in position by screws 50 which are inserted through the shafts and have their upper ends screwed into the cap. The rollers are rotatably mounted on their associated shafts 48 by means of plain bearings 51 and are sealed at opposite ends by plastics bushes 52 which rotate in contact with the upper and lower flanges of the cage.

Formed at the lower end of the outwardly tapered part 45 of the hub 31 is the male part 53 of the bayonet fastening for attaching the pump head to the housing. Fastened to the upper side of the male bayonet part 53 by screws 55 is an annular guard support 56 carrying an arcuate guard 57 which projects upwardly adjacent the outside of the head to shield the rollers 32 disengaged from the resilient tube 25 against accidentally contact with a pump operator (see also Figs. 1 and 2). The guard 57 has an operating handle 58 secured to the guard by screws 59. When the head is assembled to the housing, the hub is fitted over the tapered boss 29 so that the driving blade 41 engages in the slot 40 in the input shaft 39 and the guard is manipulated so as to align the flanges of the male bayonet part with cooperating recesses in the inner periphery of the female bayonet ring 27, whereupon the male part will drop into these recesses and the annular guard support 56 seats on the top of the female bayonet ring. Thereafter, the guard support can be turned by means of the handle 58 so as to engage the bayonet fastening, and the arrangement is such that, when the fastening is fully engaged, the guard 57 is in the correct position for shielding the disengaged rollers.

The paediatric head illustrated in Fig. 7 is constructed similarly to the standard head shown in Fig. 6, except that the former incorporates a planetary reduction gear mechanism for reducing the speed of rotation of the roller cage 30 and increasing the torque. Parts in Fig. 7 similar to those of Fig. 6 are indicated with like reference numerals. In the paediatric head illustrated in Fig. 7, the cage cap 60 is heightened and constructed as a hollow member having an internal cavity 61 for containing the reduction gear mechanism 62, and the input shaft 63 for coupling the head to the drive shaft 23 is formed separately from the cap and is coupled thereto via the gear mechanism 62. The hollow hub 31 is constructed in two parts, the lower tapered part 45 being connected to a central barrel portion 64 by screws 65. At its upper end, the barrel portion 64 of the hub is stepped outwardly and projects into the cavity 61 in the cap, where an internally toothed stationary gear ring 65 is coaxially fastened to its end face by screws and dowel pins 66,67. A driven gear ring 68 is fastened to the interior of the cap above and coaxially with the gear ring 65 by screws 69. The input shaft 63 is rotatably mounted within the barrel portion 64 of the hub by plain bearings 70 and is secured in position at its lower end by circlips and washers 71. At its upper end, the shaft 63 has a head 72 with an outwardly stepped periphery generally complementary to the internally stepped upper end of the hub portion 64 and this head bears on an internal shoulder of the hub via a thrust washer 73. Projecting upwardly from the head 72 of the input shaft, eccentrically with respect to its axis, is a gear shaft 74. The lower end of the gear shaft is screwed into the head. Mounted on this shaft so as to be freely rotatable thereon is a double pinion member, of which the lower pinion 75 has more teeth than the upper pinion 76. The lower pinion meshes with the stationary gear ring 65 whilst the upper pinion meshes with the driven gear ring 68 attached to the cap. Rotation of the input shaft 63 rotates the shaft 74 about the axis of the shaft 63, whereupon the pinion member 75,76 is rotated by engagement of the pinion 75 with the stationary gear ring 65. Resulting rotation of the pinion 76 meshing with the gear ring 68 attached to the cap produces rotation of the latter and, hence, rotation of the roller cage.

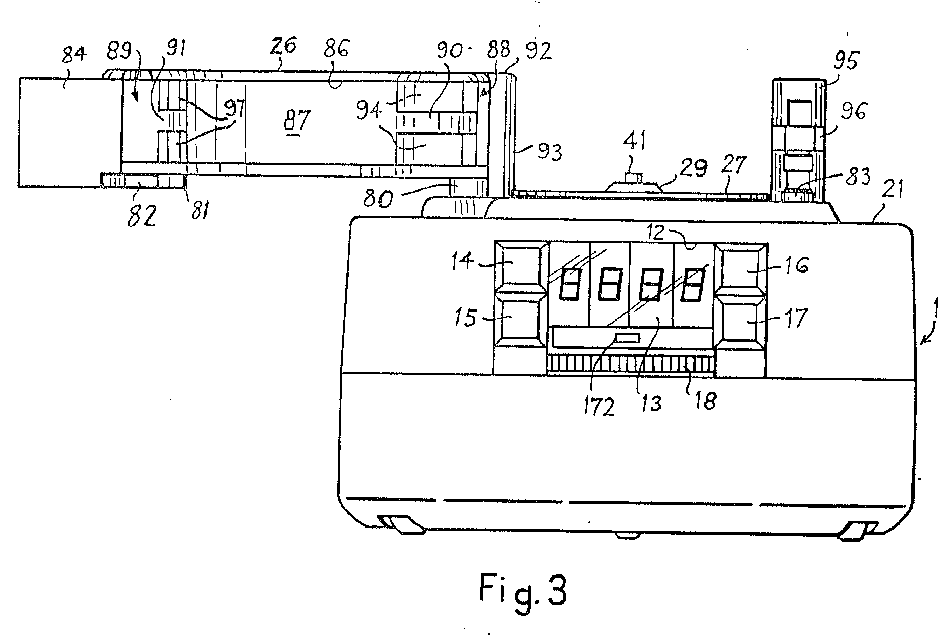

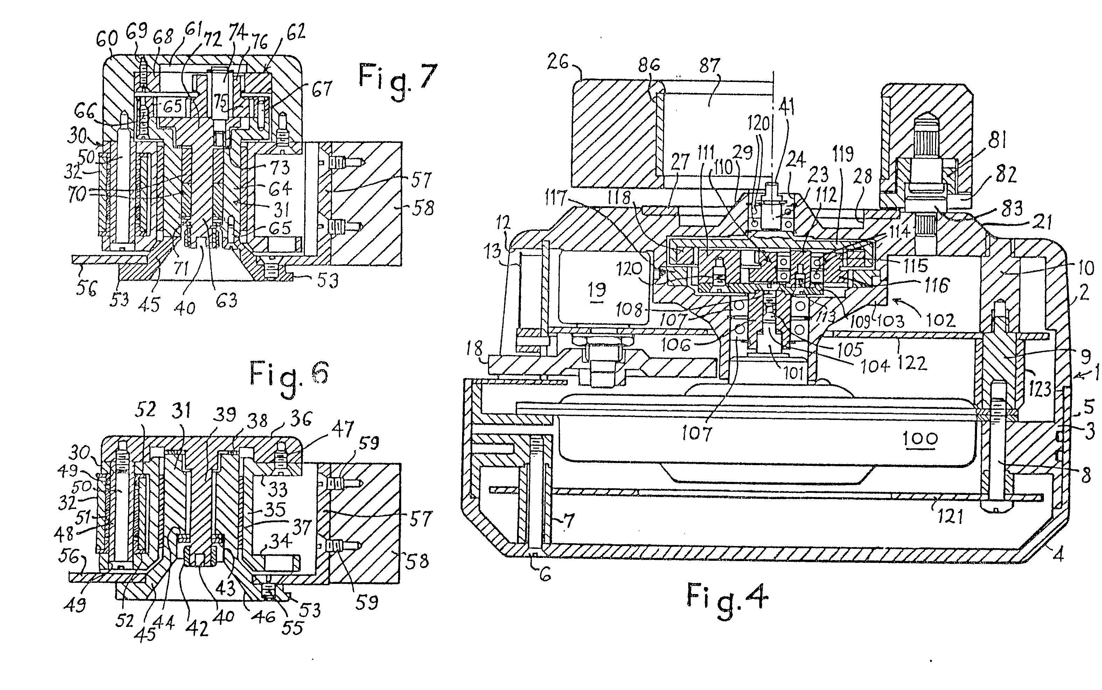

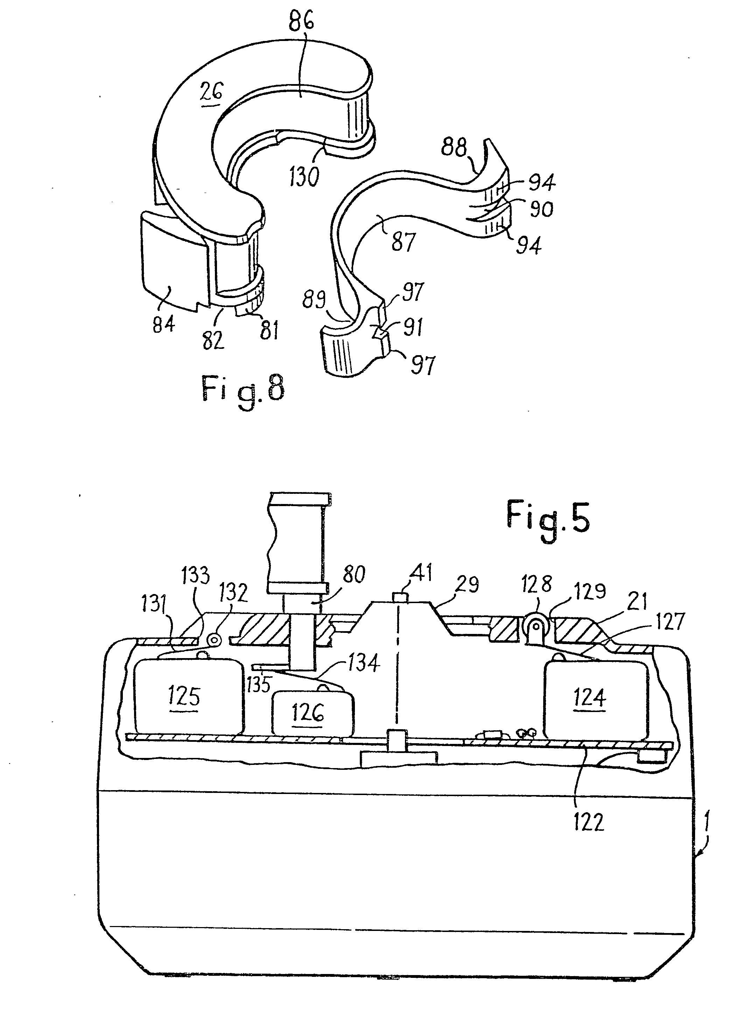

Referring again to Figs. 1 to 4, pivotally mounted on the top 21 of the pump housing adjacent the head 22 is the arcuate tube retainer 26 for urging the pump tube 25 (Fig. 1), which is made from a resilient, transparent, synthetic material, into cooperation with the head. The retainer 26 is of generally semi-circular shape in plan and may be moulded from plastics material, such as nylon. It is pivoted adjacent one end, the input side of the pump, on a pin 80 projecting upwardly from the housing top and is swingable between an open position (shown in Figs. 2 and 3), in which the opposite, discharge end of the retainer is spaced from the pump head, and a closed position (shown in Figs. 1 and 4) in which the retainer is concentric with the head. At its discharge end, the retainer 26 has a pivoted latch member 81 provided with a hook-like catch portion 82 which, when the retainer is in its closed position, is engageable with a detent 83 disposed on the housing top and can be turned to lock the retainer in this closed position. In addition to locking the retainer, the latch member 81 and detent 83 also serve to index the retainer in a precisely predetermined closed position relative to the head 22. The latch member is turnable by an integral finger lever portion 84 which, in the locked position of the retainer, is received within a cavity 85 formed in the outer periphery of the retainer so that it is generally flush with the outer periphery of the retainer, thereby to reduce the risk of accidental opening. In its inner periphery (see also Fig. 8), the retainer has a channel 86 of rectangular cross-section which receives an arcuate liner 87 moulded from nylon or other plastics material and defining an arcuate surface for engaging and urging the tube 25 against the rollers 32. When the retainer is closed, this arcuate surface is disposed concentrically with respect to the head and spaced a small predetermined radial distance from the path of movement of the rollers. The main body of this liner is of rectangular cross-section. The channel 86 extends about opposite, radiused ends of the retainer and the liner includes outwardly curved end portions 88,89 engaged in the radiused end portions of the channel. The liner end portions 88,89 are formed with grooves or notches 90,91 for engaging and gripping the pump tube 25. The liner 87 is an interference fit in the channel but can readily be removed from the retainer by manually lifting either of the liner end portions 88,89 from the channel.

Projecting upwardly from the housing top 21 adjacent the pivoted, input end of the retainer is a post 92 which is sheathed in a plastics sleeve 93 and which cooperates with the adjacent notch 90 of the liner in order to grip the tube 25. The width of the notch 90 is slightly smaller than the external diameter of the tube 25 and, when the retainer is closed, the curved lands 94 on opposite sides of the notch engage the plastics sleeve 93 sheathing the post so that the tube is urged into the notch 90 and slightly distorted, whereby the tube is securely gripped between the notch and the post, and this gripping action resists longitudinal displacement or slippage of the tube between the head and the retainer. Projecting upwardly from the housing top adjacent the locking detent 83 is a monitoring post 95 having a monitoring device for monitoring the contents of the transparent tube 25. The post 95 has a generally rectangular monitoring slot 96 in its side adjacent the retainer and, when the retainer is in its

-14-

closed position, the tube 25 leads from between the pump
head and the retainer via the slot 96. Terminating in
the top and bottom sides of the slot, respectively, are
the ends of optical fibers, the opposite ends of which
are optically associated with a light source and a photodetector device mounted within the housing. At this end
of the retainer, the notch 91 is of similar width to the
notch 90 but the lands 97 defining the notch 91 are foreshortened so that the end portion 89 of the liner urges
the tube into the monitoring slot 96 when the retainer is
closed. However, the post 95 also urges the tube into the
adjacent notch 91 so that the tube is also gripped at this
side of the pump to resist displacement of the tube. The
monitoring slot faces towards the pump head and its axis
is inclined at approximately $45^{\circ}$ to the radial direction
so as to facilitate exit of the tube from between the
retainer and head and prevent kinking of the tube. When
a double tube and appropriate liner 87 are used with the
pump, such as to provide for simultaneous supply and discharge of blood to a patient, the arrangement is such that
the supply tube can be led through the monitoring slot
whilst the discharge tube exits between the post 95 and
the pump head.

In order to fit the pump tube 25 about the pump head
22, the tube retainer 26 is swung into its open position,
as shown in Fig. 2, and the tube is threaded between the
post 92 and the adjacent end portion 88 of the liner,
about part of the circumference of the pump head, and
through the monitoring slot 96 in the post 95. Thereafter,
the retainer is closed so as to clamp the tube against the
rollers 32 of the head, which flatten the tube where they
are in engagement with the latter, and it is locked in
this closed position by turning the latch member 81 into
its locking position by means of the finger lever 84.
When the retainer is locked in its closed position, the
tube 25 is gripped by the notches 90,91 at opposite ends of the retainer and this effectively prevents longitudinal displacement of the tube. When the retainer is closed, the rollers at the side of the head embraced by the retainer are shielded by the latter whilst, at the opposite side of the head, they are shielded by the arcuate guard 57 so that the rollers are not exposed and cannot be accidentally contacted by a person, thereby risking injury.

The liner 87 is adapted to urge a tube 25 of predetermined diameter into operative engagement with the head, when the retainer is locked in its closed position, so that as the head 22 rotates, the resilient tube is compressed at spaced positions by the rollers, which compressed positions are successively advanced along the portion of the tube clamped between the retainer and the head, thereby to pump a liquid along the tube by peristaltic action. If it is desired to change the range over which the pumping rate is adjustable by variation of the speed of rotation of the pump head, thereby necessitating the use of a tube 25 of different diameter and, consequently, a different liner 87 for urging the different diameter tube into correct cooperation with the rollers of the head, the fitted liner is readily removable from the retainer 86 and is interchangeable with the appropriate new liner for the different tube.

Referring more particularly to Fig. 4, the motive power for the pump is provided by a DC printed circuit motor 100 mounted within the housing 1. The motor is mounted on the central metal part 3 of the housing by the screws 8 and the studs 9. The armature shaft 101 of the motor is coupled to the drive shaft 23 by means of a planetary reduction gear mechanism 102 housed within a plastics gear case 103 fastened to the underneath of the top part 2 of the housing. The armature shaft projects into the lower end of the gear case and has a driving blade 104 at its upper end which engages in a cooperating slot 105 in the adjacent end of a stub shaft 106 which is rotatably mounted in the gear case, coaxially with the armature shaft, on ball bearings 107. Secured to a squared upper end of the stub shaft by means of a screw 108 is a gear carrier plate 109 for a planetary gear member 110 and a counterweight 111 which are mounted on the plate eccentrically with respect to the stub shaft 106. The counterweight is fastened to the plate by a screw 120. The planetary gear member is a double pinion member which is freely rotatable on a gear shaft 112 fastened to the carrier plate by a screw 113. It is mounted on the shaft 112 by means of ball bearings 114. It comprises two pinion wheels 115,116, of which the lower pinion 116 has more teeth than the upper pinion. The lower pinion meshes with an internally toothed gear ring 117 fixed to the gear case, whilst the upper pinion meshes with an internally toothed gear ring 118 attached to the skirt of a wheel member 119 mounting the drive shaft 23. The wheel member 119 is supported from the top part of the housing by the drive shaft 23, which is journalled in the opening 24 in ball bearings 120, and is rotatable relatively to the carrier plate 109 and the gear case 103. Rotation of the motor armature shaft 101 rotates the carrier plate, whereupon the pinion member 110 is rotated by engagement of the pinion 116 with the fixed gear ring 117. The resulting rotation of the pinion 115 meshing with the gear ring 118 attached to the wheel 119 rotates this wheel and, hence, the drive shaft 23.

Referring also to Fig. 5, mounted within the housing 1 are printed circuit boards 121,122 carrying the electrical components of the control circuitry of the pump unit. The lower printed circuit board 121 is mounted below the motor 100 by means of the screws 8. It is connected to the upper printed circuit board 122, which is of annular configuration and is mounted about the gear case 103, by suitable connecting leads and blocks (not shown). The board 122 is mounted in position by being clamped between the pegs 10 and tubular spacers 123 mounted on the studs 9. Disposed on the upper board are the speed control potentiometer 19 for the motor, the light source and photodetector associated with the monitoring post 95, two microswitches 124 for detecting the size of the liner 87 fitted into the tube retainer 26, a third, double pole microswitch 125 for prohibiting pump operation unless a pump head is properly installed on the pump and for detecting the type of head installed, a fourth microswitch 126 for prohibiting pump operation unless the tube retainer 26 is locked in its closed position and other sensing circuitry. The two microswitches 124 are equipped with levers 127 having roller followers 128 on their free ends which project through apertures 129 in the housing top so as to be engageable by one or more cam members (not shown) of a liner 87 fitted in the retainer, when the latter is moved into its closed position. Four liners are normally provided for use with each head 22 and three of the liners are fitted with cam members which actuate one or both microswitches 124 when the tube retainer is closed so that the sensing circuitry detects the size of liner and, hence, the diameter of the tube being used in the unit. Adjacent the rollers 128, the side of the retainer channel 86 is formed with an opening 130 (Figs. 2 and 8) so as to permit the liner cam members to engage with the roller followers 128. The microswitch 125 is also equipped with a lever 131 and a roller follower 132 which projects through an aperture 133 in the housing top for engagement by the guard handle 58 of a pump head. When the handle 58 is turned to actuate the bayonet fastening and lock the head to the housing, it actuates the microswitch 125 which thereby detects proper installation of the head. The microswitch 126 is equipped with a lever 134 which is actuated by an arm 135 attached to the bottom end of the pivot pin 80 of the tube retainer to detect when the retainer is closed.

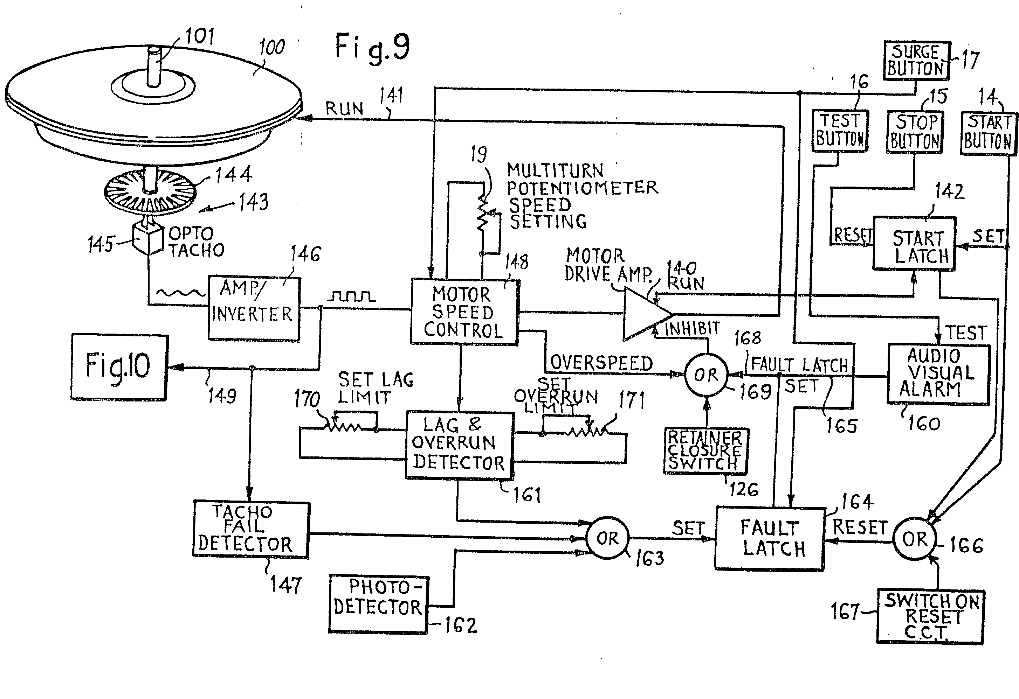

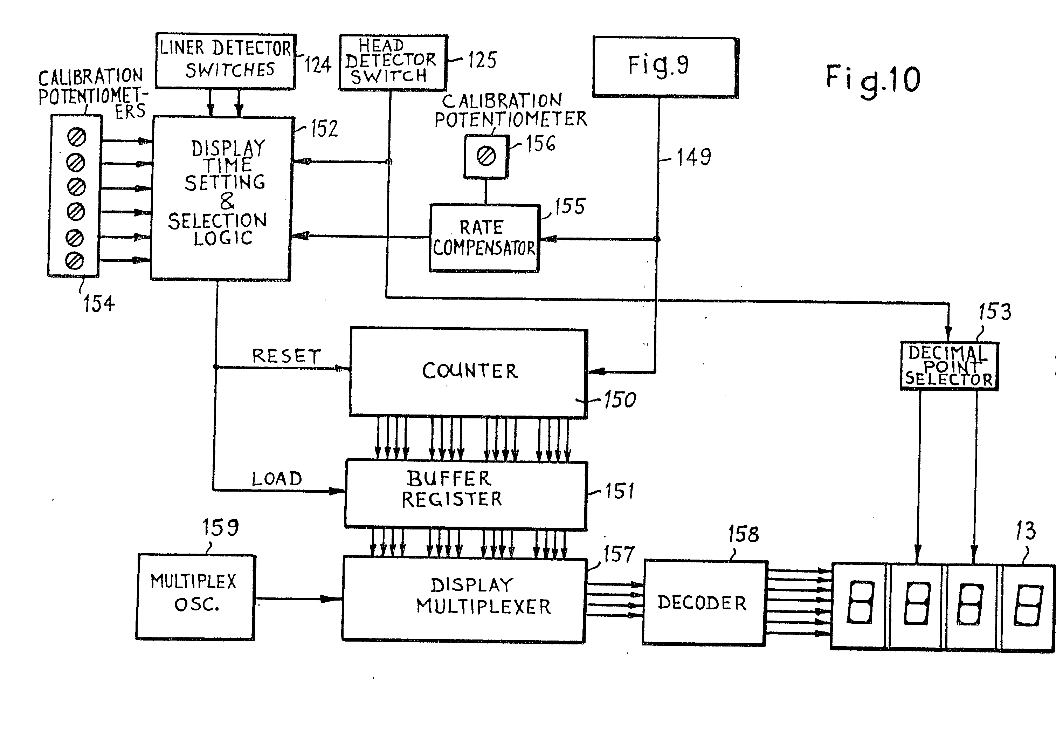

The DC printed circuit motor 100 has a very fast response time which permits operation of the motor using pulse drive techniques to control its speed, that is, by applying full power for a longer or shorter mark-space ratio depending on the load imposed on the motor and the required rotational speed. Referring to Fig. 9, electrical power, which is supplied to the control circuitry by a lead 137 (Figs. 1 and 2) for example from a 12 v DC source, is applied to the motor 100 via an amplifier 140 which is connected to the motor by a line 141. The amplifier is switched on or off by actuation of the start and stop buttons 14,15 which control the amplifier via a start latch circuit 142. Rotation of the motor is detected by means of a photo-tachometer device 143 directly coupled to the armature of the motor. In a preferred embodiment, the motor has one hundred and seventeen commutation periods per revolution which permits smooth operation of the motor at low motor speeds and with a negligible cogging effect, and so as to avoid the possibility of hunting at low speeds, the tachometer disc 144 driven by the motor armature shaft has a number of divisions which has a common denominator with the number of commutation periods and is twenty-six divisions per armature revolution. The signal produced by the photodetector 145 of the tachometer is fed to an amplifying and inverting circuit 146 and is in the form of a sine wave of approximately one volt peak-to-peak with a frequency ranging from 400 Hz to 15 KHz over the full operational speed range of the motor. This sine wave signal is amplified by the amplifier of 146 which has inputs arranged so that the reference voltage is self-centering on the mean r.m.s. value of the incoming tachometer signal, thereby automatically compensating for any variations in the level of the tachometer signal due to speed or temperature variations. The output from the amplifier in circuit 146 is a square wave with an amplitude of approximately 8 volts. This is AC coupled to an inverter which is used to produce a short negative pulse from the leading edge of each positive pulse of the amplifier output signal, and the output from the inverter is supplied to a tachometer fail detector circuit 147, a motor speed control circuit 148 and, via a line 149, to the digital display control circuit of Fig. 10.

The rotational speed of the motor 100 is regulated by discharging a control capacitor in the motor speed control circuit 148 at a controlled rate, the charge rate of which capacitor can be varied by means of the multi-turn speed control potentiometer 19. The motor drive amplifier 140 is arranged to switch-on each time the voltage in the control capacitor reaches a pre-set reference level. Thus, when the speed control potentiometer 19 is set to its minimum resistance, the control capacitor charges at its maximum rate, as determined by a limiting resistor arranged to set the maximum permissible speed, and the motor switches-on for a longer period and, hence, rotates faster until the discharge rate of the capacitor, as determined by the tachometer device 143, equals the charge rate determined by the setting of the potentiometer 19 and the associated limiting resistor, whereupon the circuit is in balance and the motor is running at its maximum permissible speed. If the speed control potentiometer 19 is then turned to a higher resistance by turning the control wheel 18 (Figs. 3 and 4), the motor speed causes the tachometer to discharge the capacitor faster than it can be charged and power is therefore removed from the motor so that the pump load causes the motor to slow down until an equilibrium is again reached, whereupon the motor is pulsed on and off, as is necessary, to maintain the speed at the selected setting. This method of speed control ensures that the power applied to the motor automatically compensates for changes in load and maintains a substantially constant speed at any selected speed setting over the whole speed range.

The digital display panel 13 is a four-digit, decimal, seven-segment, l.e.d. (light emitting diode) display panel for indicating the flow rate in millilitres per minute by increments of 0.1 millilitres over a range from 5 to 150 millilitres per minute, when the standard pump head 22 is installed on the unit, and by increments of 0.01 millilitres per minute over a range of from 0.50 to 15.0 millilitres per minute, when the pump is fitted with a 10:1 reduction or paediatric pump head. The flow rate is measured from the rotational speed of the motor using the output pulses from the amplifier/inverter circuit 146 to drive a four decade b.c.d. (binary coded decimal) counter 150 (Fig. 10) coupled to a display buffer register 151 to which the total count over a predetermined counting period is transferred. The counting period is regulated by a precision time setting and selection logic 152 connected to control the counter 150 and the buffer 151. Hence, the number appearing on the display panel 13 is the number of pulses occurring during a period determined by the setting of the logic 152 and, by changing this counting period, it is possible to produce a digital indication of the flow rate for more than one tube diameter. In this embodiment, the requirement is to indicate the flow rate for three different diameter tubes or liners used with the standard pump head 12 and a further three different diameter tubes with the paediatric head, and this is achieved by controlling the timing logic 152 in response to actuation of the liner detecting microswitches 124 and the head detecting microswitch 125. The two microswitches 124 are arranged to be operated by the liners in a binary fashion to identify the tube being used and to set the timing logic 152 according to the tube diameter. The microswitch 125 signals that the head installed on the pump unit is a standard or paediatric head and cooperates with the signals produced by the liner microswitch 124 to select the correct timing circuit and to position the decimal/point indication on the display panel 13 via the decimal point selection circuit 153. Hence, the timing logic is automatically set in accordance with the tube diameter and type of head and this reduces the possibility of human error in selecting the correct flow rate control for the different tube diameters and heads.

Each of the individual timing circuits in the timing logic 152 may be calibrated to permit accurate setting of the display for each tube diameter and flow rate possibility by six pre-settable potentiometers 154 connected to the individual timing circuits and mounted on the underneath of the lower circuit board 121 (Fig. 4) so as to be accessible from the bottom of the housing 1.

The timing logic produces a linear indication of flow rate based on the rotational speed of the motor 100. However, in practice, the flow rate achieved does not vary in a linear relationship to the rotational speed of the motor. The efficiency of the pump increases as the rotational speed of the pump head 22 increases. It is therefore necessary to incorporate a speed compensation factor using a rate compensation circuit 155 operated from the tachometer pulses on the line 149 and which reduces the time period defined by the individual timing circuits in the timing logic 152 by a pre-settable amount based on the pumping rate of the unit. This rate compensation circuit can also be calibrated by means of a pre-settable potentiometer 156 mounted on the underneath of the lower circuit board 121 so as also to be accessible from the bottom of the housing.

The outputs from the buffer register 151 are distributed to the digital display panel 13 by a display
multiplexer 157 and a b.c.d. to seven segment decoder
158. The display multiplexer enables the interconnections
between the display panel and the buffer register 151 to
be reduced. The multiplexer successively scans and decodes the b.c.d. data stored in the register under the
control of the multiplex oscillator 159 and actuates the
four sections of the display panel 13 sequentially and
one character at a time at a rate which is undetectable
to the human eye, thereby to produce an essentially instantaneous digital display of the liquid flow rate through
the pump.

Several fault detection or safety circuits are provided in the control circuitry to monitor correct operation of the pump unit and to produce a warning signal to
an operator by means of an audio-visual alarm 160, when a
fault occurs in the unit or there is an irregularity in
the liquid being pumped through the unit. The safety circuits include the tachometer fail detector 147, a lag and
overrun detector 161 responsive to the motor speed control
circuit 148, and the photodetector 162 associated with the
monitoring post 95, all connected to the three inputs of
an OR gate 163 having its output connected to a fault latch
circuit 164 adapted to actuate the audio-visual alarm 160
via the line 165. The fault latch 164 may be reset in
response to operation of the stop button 15 which is
arranged to reset the start latch 142 which, in turn, is
connected to one input of an OR gate 166 having its output
connected to the reset input of the fault latch 164. The
start button 14 is connected to a second input of the OR
gate 166 and a reset circuit 167, which inhibits operation
of the fault latch during an initial switch-on period, for
example, five seconds, when the pump is switched-on, is
connected to a third input of this OR gate 166.

The output of the fault latch 164 in addition to being connected to the audio-visual alarm 160, via the line 165, is also connected, via a line 168, to one input of an OR gate 169 having its output connected to the motor drive amplifier 140 for supplying an inhibiting signal to this amplifier and inhibiting operation of the motor upon occurrence of a fault. The OR gate 169 also has inputs connected to an output of the motor speed control circuit 148, which supplies a signal to the OR gate in the event of overspeeding of the motor, and to the retainer position microswitch 126 which applies a signal to the OR gate unless the tube retainer 26 is locked in its closed position.

In order to check for the correct functioning of the audio-visual alarm and the digital display, the test button 16 may be depressed at any time, whether the pump unit is operating or not, without interfering with the normal operation of the unit. The test button will then cause all the segments of each section of the display panel 13 to be illuminated and the audio-visual alarm to be actuated for as long as the test button remains depressed.

Any of the fault-detection circuits described above will trigger the fault latch 164 which, in turn, switches off the motor power and actuates the audio-visual alarm 160. Once operated, the fault latch 164 will remain in this condition until it is reset by depression of the stop button 15, or is over-ridden by the start button 14 in order to permit continued operation of the pump unit, if necessary. The start button 14, which is connected to the second input of the OR gate 166, is arranged to override the fault latch 164 in the event of a fault and whilst the start button remains depressed. Another safety feature is the surge button 17 which permits maximum pumping rate to be selected substantially instantaneously. To this end, the surge button is connected to switch the motor speed control circuit 148 for maximum speed and over-

-24-

ride the fault latch 164, when depressed.

The various fault detection circuits operate as follows:-

(A)  Photo-tachometer Failure

A tuned R-C circuit in the tachometer fail detector circuit 147 monitors the presence of the tachometer pulses at the output of the amplifier/inverter circuit 146 and, if the time interval between these pulses exceeds a predetermined minimum interval, when the motor 100 is switched-on, then the detector circuit 147 applies a signal to the OR gate 163 so as to signal the fault latch 164, whereupon the motor is switched-off and the alarm 160 is actuated. The tachometer fail circuit detects a mechanical failure of the motor or an electrical failure in the motor drive logic.

(b)  Excessive Rate Changes in Motor Speed

If the motor speed starts to increase or decrease at a rate in excess of predetermined limits, as detected by the DC level generated on the input to the motor drive amplifier 140 from the speed control and tachometer circuits, then either of two independently pre-settable limit detectors in the circuit 161 and defining the upper and lower permitted limits of the rate of change of the motor speed applies a signal to the input of the OR gate 163 to trigger the fault latch 164.  The limit detectors can be preset to the required limits by two potentiometers 170,171 mounted on the underneath of the lower circuit board 121 (Fig. 4) and adjustable from the bottom of the housing 1.

(c)  Motor Drive Transistor Failure

To protect against a short circuit failure of the motor drive transistor in the amplifier 140, a second transistor is connected in series with this motor drive transistor and is controlled by the start latch 142 such that it is triggered by depression of the start button 14 and reset by depression of the stop button 15, unless over-ridden by the fault latch 164. This second transistor, therefore, normally conducts when the start latch is triggered. However, if the drive transistor fails to a short circuit condition on the collector-emitter junction, the motor 100 would normally run away uncontrolled to maximum speed. If this occurs, the rapidly increasing pulse train produced by the photo-tachometer 143 causes the rate change detector circuit 161 to trigger the fault latch 164 which, in turn, over-rides the start latch and switches-off the second transistor. The fault latch is reset by operation of the stop button, but only when the fault condition is removed.

Whilst it is necessary to permit continued operation of the pump unit in the event of a fault and under certain circumstances and this is provided for by continuous depression of the start button, the safety feature described in the preceding paragraph prevents uncontrolled operation of the motor 100 due to a failure of the motor drive transistor, even when the fault latch 164 is over-ridden by depression of the start button.

(d) Tube Retainer Closure Detector

The microswitch 126 grounds the base of the second or auxiliary motor drive transistor to prevent operation of the motor if the tube holder 26 is not closed, thus prohibiting the operator from actuating the motor and rotating the pump head whilst access to the rollers 32 is possible and constitutes a physical hazard to the operator.

(e) Liquid Photo-scanning Device

The photodetector 162 senses a change in light transmission through the tube 25 and the liquid flowing therein and actuates the fault latch 164 in the event of a sudden change in the level of the light beam projected through the tube, thereby enabling clots, bubbles, or other foreign bodies in suspension in the liquid being pumped to be detected, whereupon the motor 100 is switched-off and the alarm 160 is actuated to prevent further pumping until the cause has been analysed.

The audio-visual alarm 160 comprises a 400 Hz peizo-electric crystal alarm modulated at a frequency of 2 Hz to provide an audible signal, and a warning light 172 (Fig. 3) which is a red light emitting diode which is normally off and, on being actuated by an alarm condition, flashes red at a frequency of 2 Hz.

Whilst a particular embodiment has been described, it will be understood that various modifications can be made without departing from the spirit and scope of the invention as defined by the appended claims.

## CLAIMS

1. A peristaltic pump unit comprising a rotatable pump head (22) including a plurality of rollers (32) rotatable about axes substantially parallel to the axis of rotation of the head, a drive mechanism (100,102) for rotating the head, and a tube retainer (26) having an arcuate surface closely adjacent and generally concentric with the head (22) for urging a resilient pump tube (25) into operative engagement with the head, whereby rotation of the head successively rotates the rollers into compressive engagement with the pump tube to pump fluid therealong, characterized in that the tube retainer (26) is pivotally mounted adjacent one end of its arcuate surface and is swingable between an open position, in which the pump tube (25) is releasable from the head (22), and a predetermined closed position, in which the arcuate surface urges the pump tube into operative engagement with the pump head, and further characterized by means (81,83) for indexing and locking the retainer in its predetermined closed position.

2. A pump unit according to claim 1, characterized in that the indexing and locking means comprises a latch device (81,83) disposed adjacent the end of the arcuate surface opposite to the pivot mounting (80) of the retainer.

3. A pump unit according to claim 2, characterized in that the latch device comprises a pivoted latch member (81) mounted on the retainer (26) and a cooperating detent (83) disposed adjacent the head (22) in a predetermined position.

4. A pump unit according to claim 1, 2 or 3, characterized in that the arcuate surface is defined by a removable liner (87) for urging a pump tube (25) of predetermined diameter into operative engagement with the head (22), said liner being mounted in the retainer and being interchangeable with at least one other liner for use with a

-28-

pump tube (25) of different predetermined diameter.

5.    A pump unit according to any preceding claim, characterized in that the arcuate surface has a notch (90) of a predetermined width smaller than the diameter of the pump tube (25) located adjacent at least the end thereof disposed at the input side of the pump, and means (92) is provided for urging the pump tube into engagement with the notch (90) upon closing of the retainer, whereby the tube is distorted into and gripped by the notch to resist longitudinal displacement of the tube.

6.    A pump unit according to claim 5, characterized in that the or each notch (90,91) is curved radially outwardly away from the head.

7.    A pump unit according to claim 5 or 6, characterized in that the means for urging the pump tube into the or each notch (90,91) comprises a post (92,95) which bears against or is disposed closely adjacent the lands (94,97) defining the notch upon closing of the retainer (26).

8.    A pump unit according to any preceding claim, characterized in that the drive mechanism (100,102) for rotating the pump head (22) is disposed in a housing (1) having the pump head mounted thereon, said pump head being releasably fastened to the housing, whereby the head is removable and is interchangeable with another pump head.

9.    A pump unit according to claim 8, characterized in that the pump head (22) is fastened to the housing (1) by a bayonet fastening device (27,53) having one part (27) secured to the housing and the cooperating part (53) attached to the pump head, said cooperating part including an upstanding roller guard (57) which, when the cooperating part (53) is turned to engage the bayonet fastening, shields the rollers of the head on the side thereof opposite the retainer.

10.    A pump unit according to any preceding claim, characterized in that the pump head (22) comprises a hollow hub (31) fastened to the housing, a roller cage (30) containing the rollers (32) rotatably mounted on the hub, an input shaft (39,63) rotatably mounted in the hub for coupling to the drive mechanism (100,102), and a cap (36,62) extending radially over the roller cage (30) and connecting the input shaft to the roller cage.

11. A pump unit according to claim 10, characterized in that the input shaft (63) is connected to the cap (60) by a reduction gear mechanism (62), preferably, a planetary reduction gear mechanism.

12. A pump unit according to any preceding claim, characterized in that the pump head (22) has five rollers (32) equally spaced about the head within a circle having a diameter not greater than 63.5 mm.

13. A pump unit according to any preceding claim, characterized in that the drive mechanism comprises a DC printed circuit motor (100) coupled to a drive shaft (23) connected to the pump head (22) by reduction gear mechanism (102), preferably a planetary reduction gear mechanism.

14. A pump unit according to any preceding claim, characterized by electrical control circuitry including means (19,148) for adjusting the speed of the drive mechanism (100,102), and means (13) for providing a visual indication of the pumping rate at the selected speed of the drive mechanism, said visual indication being derived from a signal identifying the speed of rotation of the drive mechanism and another signal identifying the diameter of the pump tube (25) cooperating with the head.

15. A pump unit according to claim 14, characterized in that the signal identifying the rotational speed of the drive mechanism is produced by tachometer means (143) for sensing the rotational speed of the motor, and the tube identifying signal is produced by means (124) for sensing the selected liner fitted into the tube retainer (26).

16. A pump unit according to any preceding claim, characterized by electrical control circuitry comprising fault-detection means (147,161,162) for monitoring the operation of the pump, and means (160,164) responsive to the fault-detection means for producing a warning signal and/or switching-off the drive mechanism.

17.    A pump unit according to claim 16, characterized in that the fault-detection means comprises a photodetector device (162) arranged to scan the fluid flow through the pump tube (25) at the discharge side of the pump in order to detect bubbles or other foreign matter, whereupon to actuate the fault-detection responsive means (160,164).

18.    A pump unit according to claim 16 or 17 as appendant to claim 15, characterized in that the fault-detection means comprises a tachometer fail detector (147) and a detector 161 for sensing overspeeding or lag of the motor, and further characterized by means (126) for inhibiting operation of the motor unless the tube retainer (26) is disposed in its closed position.

19.    A pump unit according to claim 16, 17 or 18, characterized by first manually operated means (14) for overriding operation of the fault-detection responsive means upon occurrence of a fault condition, and second manually operated means (17) for substantially instantaneously switching the drive mechanism to full speed and inhibiting operation of the fault-detection responsive means.

Fig.1

Fig.2

Fig. 3

Fig. 7

Fig. 6

Fig. 4

3/6

0033666

Fig. 8

Fig. 5

Fig.9

Fig.10

Fig.9

CALIBRATION POTENTIOMETERS

LINER DETECTOR SWITCHES — 124

HEAD DETECTOR SWITCH — 125

CALIBRATION POTENTIOMETER — 156

DISPLAY TIME SETTING & SELECTION LOGIC — 152

RATE COMPENSATOR — 155

— 149

— 154

RESET

COUNTER — 150

LOAD

BUFFER REGISTER — 151

DECIMAL POINT SELECTOR — 153

MULTIPLEX OSC. — 159

DISPLAY MULTIPLEXER — 157

DECODER — 158

13

9/9

0033666

European Patent
Office

**EUROPEAN SEARCH REPORT**

Application number

EP 81 30 0474

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | F 04 B 43/12 |
| X | US - A - 4 138 205 (WALLACH) <br><br> * Column 3, lines 7-64; column 5, lines 24-27; figures 1-4 * | 1-3,5, 6 | |
| X | FR - A - 2 262 209 (LAUTERJUNG) <br><br> * Page 9, lines 7-14; page 10, lines 1-8; page 12, lines 36-40; page 13, lines 1-24; page 14, lines 20-31; figures 1-12 * | 1,2,13, 14,15, 16,17 | |
| X | US - A - 3 927 955 (SPINOSA) <br><br> * Column 4, lines 35-46; column 5, lines 1-29; column 8, lines 10-26; figures 2,3 and 9 * | 1,2,8, 14,18 | TECHNICAL FIELDS SEARCHED (Int. Cl.³) <br><br> F 04 B <br> A 61 M |
| X | GB - A - 1 353 156 (GILLESPIE) <br><br> * Page 1, lines 45-96; page 2, lines 1-14; figures 1 and 2 * | 1,2,7, 8,13, 14 | |
| | US - A - 4 184 510 (MURRY) <br><br> * Column 16, lines 62-66; column 17, lines 28-67; figures 8,9 * | 1,12 | |
| | US - A - 3 762 836 (DE VRIES) <br><br> * Column 1, lines 1-9; column 2, lines 40-57; figures 1-4 * | 4,8 | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | US - A - 1 988 337 (SANTIAGO) <br><br> * Page 1, right-hand column, lines 5-16; page 2, right-hand column, lines 11-19; figures 1-3 * <br><br> ./.. | 4 | |
| The present search report has been drawn up for all claims | | | &: member of the same patent family, corresponding document |

| Place of search | Date of completion of the search | Examiner |
|---|---|---|
| The Hague | 29.04.1981 | HEINLEIN |

EPO Form 1503.1   06.78

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | <u>US - A - 3 353 491</u> (BASTIAN)<br><br>* Column 3, lines 19-25; figure 1 *<br><br>--- | 5,6 | |
| | VOEDINGSMIDDELENTECHNOLOGIE, vol. 4, no. 14, 4th April, 1973, page 17,<br>"Slangenpomp met vergroot capaciteitsbereik"<br><br>* Entire article"<br>--- | 8,14, 15 | |
| | <u>FR - A - 2 321 147</u> (FRESENIUS)<br><br>* Page 2, lines 31-33; page 3, lines 17-31; page 4, lines 7-23; figures 5 and 6. *<br>& GB - A - 1 519 415<br>--- | 8,13, 14,18 | TECHNICAL FIELDS SEARCHED (Int. Cl.³) |
| | <u>DE - A - 2 033 344</u> (INSTITUT FÜR TECHNOLOGIE)<br><br>* Page 4, paragraph 2; figure * | 13 | |
| | ---------- | | |

EPO Form 1503.2   06.78